(19) Europäisches Patentamt
European Patent Office
Office européen des brevets

(11) **EP 4 046 562 A1**

(12) **EUROPEAN PATENT APPLICATION**
published in accordance with Art. 153(4) EPC

(43) Date of publication:
**24.08.2022 Bulletin 2022/34**

(21) Application number: **19949195.2**

(22) Date of filing: **15.10.2019**

(51) International Patent Classification (IPC):
**A61B 1/00** (2006.01)    **G02B 13/00** (2006.01)

(52) Cooperative Patent Classification (CPC):
**A61B 1/00; G02B 13/00**

(86) International application number:
**PCT/CN2019/111324**

(87) International publication number:
**WO 2021/072656 (22.04.2021 Gazette 2021/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Shenzhen Mindray Bio-Medical
Electronics Co., Ltd.
Shenzhen, Guangdong 518057 (CN)**

(72) Inventors:
• YUAN, Xiaowen
  Shenzhen, Guangdong 518057 (CN)
• LEI, Fang
  Shenzhen, Guangdong 518057 (CN)
• FAN, Rui
  Shenzhen, Guangdong 518057 (CN)
• YANG, Li
  Shenzhen, Guangdong 518057 (CN)

(74) Representative: **KIPA AB
Drottninggatan 11
252 21 Helsingborg (SE)**

(54) **ENDOSCOPE AND ROD LENS SYSTEM THEREOF**

(57)    Disclosed are an endoscope (20) and a rod lens system (10) thereof. The rod lens system (10) comprises a first lens group (11), a diaphragm (12), and a second lens group (13). The first lens group (11) and the second lens group (13) are identical and arranged symmetrically with respect to the diaphragm (12); and the first lens group (11) and the second lens group (13) each comprise a first lens (111), a second lens (112), and a third lens (113) which are fixedly connected to each other, wherein the first lens (111) is a long-rod lens with positive focal power, the second lens (112) has positive focal power, and the third lens (113) has negative focal power.

FIG.14

EP 4 046 562 A1

**Description**

TECHNICAL FIELD

The disclosure relates to the technical field of optical systems, and more particularly to an endoscope and a rod lens system thereof.

BACKGROUND

At present, endoscopy, as a medical device, has been more and more widely used, especially in minimally invasive surgery. In addition, intravenous contrast agent (ICG) is often used for near-infrared imaging in hard mirror surgery, so as to identify the tissue structure during the surgery. In order to support fluorescence imaging, it is necessary to provide an endoscope that can clearly image the conventional image of white light and the fluorescence image of near-infrared light at the same time.

However, at present, the rod lens system in the endoscope on the market cannot correct the axial chromatic aberration of near-infrared light and the axial chromatic aberration of white light at the same time, because the general endoscope has multiple groups of rod lens systems, and the axial chromatic aberration will be magnified exponentially. Finally, the infrared light images and the white light images cannot be clear at the same time during the surgery, so can only be observed at different times. That is, it is necessary to refocus when switching between infrared and white light images. In order to correct the axial chromatic aberration of infrared and the axial chromatic aberration of white light, the design of the existing rod lens system is too complex, and there are too many cemented lenses which always increase the eccentricity control difficulty. However, the eccentricity is closely related to the image quality of the whole lens imaging. Therefore, it is necessary to provide a wide spectrum rod lens system which has a low processing difficulty and high image quality, and is capable of imaging in both a visible light range and a near-infrared light range at the same time clearly.

SUMMARY

In this regard, the present disclosure has disclosed an endoscope and a rod lens system thereof, which is capable of imaging in both a visible light range and a near-infrared light range at the same time clearly, without requiring any adjustment of a user.

According to an aspect, a rod lens system is provided, which including a first lens group, a diaphragm and a second lens group which are successively arranged from an object side to an image side, wherein the first lens group and the second lens group are identical and arranged symmetrically with respect to the diaphragm;

the first lens group and the second lens group each includes a first lens, a second lens and a third lens, wherein the first lens is an elongated rod lens which has positive focal power, the second lens has positive focal power, and the third lens has negative focal power;
the first lens, the second lens and the third lens are fixedly connected, the first lens is farther away from the diaphragm than the second lens and the third lens, and the third lens is closer to the diaphragm than the first lens and the second lens.

According to another aspect, an endoscope is provided, which including:

an endoscope body, which includes a camera interface, a light source interface, an outer sleeve and an inner sleeve, wherein the camera interface is connected with a camera device, the light source interface is connected with a light source device, and the inner sleeve is sheathed inside the outer sleeve;
a light guide assembly, which is arranged between the outer sleeve and the inner sleeve and operable to guide a light which is generated by the light source device and received through the light source interface to an observed body;
an ocular lens assembly, which is arranged inside the inner sleeve and adjacent to the camera interface;
an objective lens assembly, which is arranged inside the inner sleeve and adjacent to an insertion end of the endoscope body; and
at least one rod lens system, which includes a first lens group, a diaphragm and a second lens group which are successively arranged from an object side to an image side, wherein the first lens group and the second lens group are identical and arranged symmetrically with respect to the diaphragm; the first lens group and the second lens group each includes a first lens, a second lens and a third lens, wherein the first lens is an elongated rod lens which has positive focal power, the second lens has positive focal power, and the third lens has negative focal power; the first lens, the second lens and the third lens are fixedly connected, the first lens is farther away from the diaphragm than the second lens and the third lens, and the third lens is closer to the diaphragm than the first lens and the second lens.

The embodiments of this disclosure have provided an endoscope and a rod lens system thereof. The rod lens system includes a first lens group and a second lens group which are identical and arranged symmetrically with respect to a diaphragm, and both lens groups include a first lens, a second lens and a third lens which are fixedly connected, wherein the first lens is an elongated rod lens which has positive focal power, the second lens has positive focal power, and the third lens has negative focal power. Accordingly, the rod lens system has a simple structure and is easy to process. Meanwhile, the chromatic aberration can be effectively reduced and clear imaging can be obtained in the visible light range and near-infrared light range at the same time, through two lens groups which are symmetrically arranged and have lens combinations with specific parameters.

It should be understood that the above general description and the following detailed description are only exemplary and explanatory and do not make any limitation of this disclosure.

BRIEF DESCRIPTION OF THE DRAWINGS

In order to explain embodiments of this disclosure or technical solutions in the prior art more clearly, the following will briefly introduce drawings required in the description for the embodiments or the prior art description. It is obvious that the drawings in the following description are only some embodiments of this disclosure. For those skilled in the art, other drawings can be obtained from these accompanying drawings without paying any creative works.

FIG. 1 is a structural diagram of a rod lens system provided in an embodiment of this disclosure.
FIG. 2 is a structural diagram of a rod lens system provided in an embodiment of this disclosure.
FIG. 3 is a schematic diagram of axial chromatic aberration of rod lens system provided in FIG. 1.
FIG. 4 is a structural diagram of a rod lens system provided in an embodiment of this disclosure.
FIG. 5 is a schematic diagram of axial chromatic aberration of rod lens system provided in FIG. 4.
FIG. 6 is a structural diagram of a rod lens system provided in an embodiment of this disclosure.
FIG. 7 is a schematic diagram of axial chromatic aberration of rod lens system provided in FIG. 6.
FIG. 8 is a structural diagram of a rod lens system provided in an embodiment of this disclosure.
FIG. 9 is a schematic diagram of axial chromatic aberration of rod lens system provided in FIG. 8.
FIG. 10 is a structural diagram of a rod lens system provided in an embodiment of this disclosure.
FIG. 11 is a schematic diagram of axial chromatic aberration of rod lens system provided in FIG.10.
FIG. 12 is a structural diagram of a rod lens system provided in an embodiment of this disclosure.
FIG. 13 is a schematic diagram of axial chromatic aberration of rod lens system provided in FIG.12.
FIG. 14 is a structural diagram of an endoscope provided in another embodiment of this disclosure.

Description of main components and symbols:

10. Rod lens system; 11. First lens group; 12. Diaphragm; 13. Second lens group; 111. First lens; 112. Second lens; 113. Third lens;
20. Endoscope; 21. Endoscope body; 211. Outer sleeve; 212. Inner sleeve; 213. Camera interface; 214. Light source interface; 22. Light guide assembly; 221. Light cone; 222. Optical fiber; 23. Ocular lens assembly; 24. Objective lens assembly.

DESCRIPTION OF THE PREFERRED EMBODIMENTS

The technical scheme in the embodiment of this disclosure will be clearly and completely described below in combination with the accompanying drawings in the embodiment of this disclosure. Obviously, the described embodiments are just some the embodiments of this disclosure, not all of the embodiments. Based on the embodiments in this disclosure, all other embodiments obtained by one skilled in the art without creative work fall into the protection scope of this disclosure.

Some embodiments of this disclosure are described in detail below in combination with the accompanying drawings. Without conflict, the following embodiments and features in the embodiments can be combined with each other.

With the development of science and medical technology, laparoscopic minimally invasive surgery has been popularized. In hard mirror surgery, the tracing of key tissues can improve the success rate of surgery. Near-infrared imaging can trace the gallbladder and lymph node structure of deeper tissues, which is conducive to the identification of morphology in routine resection surgery or tumor surgery and plays a role of accurate positioning.

The near-infrared imaging commonly used in hard mirror surgery mostly uses intravenous contrast agent (ICG). The contrast agent combines with plasma protein and globulin. And it will be quickly removed from the blood flow in the normal tissue, metabolized by the liver, excreted in bile and excreted out of the body in its original form. Therefore, there will be a short-term dye accumulation in the gallbladder, common hepatic duct or internal lymph nodes, which is conducive to the identification of tissue structure during the surgery. Secondly, because the contrast agent is infrared excitation dye and the penetration depth of infrared light is deep, it is convenient to determine the deep tissue structure and avoid the damage to key tissues during the surgery. In addition, in tumor surgery, the contrast agent can be deposited in the sentinel lymph nodes which are connected with the tumor tissue through the lymph circulation, so as to achieve the accurate positioning of the sentinel lymph node and avoid the damage to patients caused by the extensive lymph node dissection.

In order to support the fluorescence imaging, it is necessary to provide an endoscope that can clearly image the conventional image of white light (visible light) and the near-infrared fluorescence image at the same time. This kind of endoscope includes ICG hard endoscope. The following embodiment takes ICG hard endoscope as an example for detailed description.

Taking laparoscope as an example, the existing laparoscope for ICG imaging on the market mainly has two disadvantages.

(1) The rod lens system cannot correct the axial chromatic aberration of the near-infrared light and the axial chromatic aberration of the white light at the same time. Because the general laparoscope has more than three groups of rod lens image conversion systems, so the axial chromatic aberration will be multiplied, which eventually leads to the fact that the infrared light images and the white light images cannot be clear at the same time during the surgery, so can only be observed at different times, and the infrared light images and white light images should be refocused when switching.

(2) In order to correct the axial chromatic aberration of the near-infrared light and the axial chromatic aberration of the white light, the rod lens system is too complex. Chromatic aberration is one of the main aberrations affecting the optical imaging. Under the condition of certain focal power, the ability of optical lens to correct chromatic aberration mainly depends on the dispersion characteristics of glass. Because the dispersion characteristics of glass are in a discrete and discontinuous distribution, the common practice is to cement multiple glasses with different dispersion characteristics under the distribution of certain focal power to achieve the required dispersion performance. The more kinds of glasses, the less difficulty to correct the dispersion characteristics, but the higher the processing difficulty and cost. For the cemented lens, too many cemented elements would increase the difficulty of eccentricity control. In the whole laparoscope, the number of rod lens is generally more than 6, and its eccentricity is closely related to the quality of the whole laparoscope. Therefore, it is very important to effectively reduce the number of cemented elements of the rod lens system under the condition that the axial chromatic aberration satisfies the chromatic aberration tolerance.

For the clear imaging of the conventional image of the white light and the near-infrared fluorescence image of the near-infrared light at the same time, the hard endoscope should use the rod lens system which can reduce the axial chromatic aberration. Because the rod lens system of this disclosure can clearly image the conventional image of white light and the infrared fluorescence image of the near-infrared light at the same time, it is called as a wide-spectrum rod lens system.

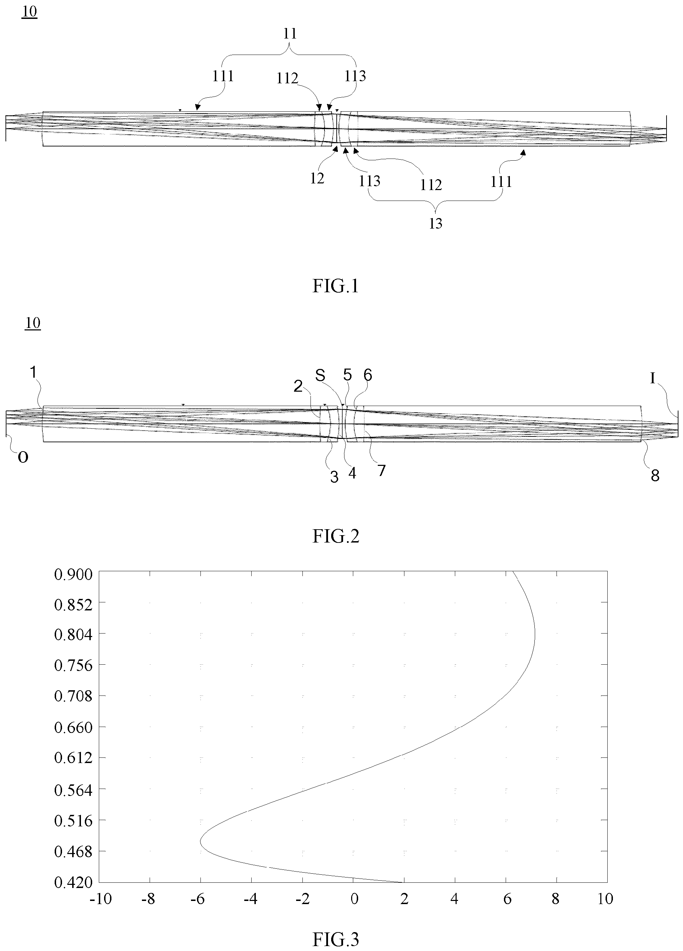

Please refer to FIG. 1, which is a structural diagram of a rod lens system provided in an embodiment of this disclosure. This rod lens system can effectively reduce the axial chromatic aberration of the visible light and the near-infrared light without any adjustment.

As shown in FIG.1, the rod lens system 10 includes a first lens group 11, a diaphragm 12 and a second lens group 13 which are successively arranged from an object side to an image side, wherein the first lens group 11 and the second lens group 13 are identical and arranged symmetrically with respect to the diaphragm 12.

Wherein the first lens group 11 and the second lens group 13 are completely identical and arranged symmetrically with respect to the diaphragm 12.

The first lens group 11 includes a first lens 111, a second lens 112 and a third lens 113, while the second lens group 12 also includes a first lens 111, a second lens 112 and a third lens 113.

Wherein the first lens 111 is an elongated rod lens which has positive focal power, the second lens 112 has positive focal power, and the third lens 113 has negative focal power.

The first lens 111, the second lens 112 and the third lens 113 are fixedly connected. Wherein in the rod lens system, the first lens 111 is farther away from the diaphragm 12 than the second lens 112 and the third lens 113, and the third lens 113 is closer to the diaphragm 12 than the first lens 111 and the second lens 112.

It should be noted that in the embodiments of this disclosure, the fixed connection between the lenses can be a cemented connection. The cemented connection is described below as an example.

Specifically, the second lens 112 is located between the first lens 111 and the third lens 113. The diaphragm 12 is located in the center of the first lens group 11 and the second lens group 13. Alternatively, the diaphragm 12 is an aperture diaphragm.

It can be understood that the first lens group 11, the diaphragm 12 and the second lens group 13 are arranged successively along an optical axis from the object side to the image side.

It should be noted that the diameters of the first lens 111, the second lens 112 and the third lens 113 can be identical in some embodiments, but can also be different in some embodiments. In practical application, the first lens 111, the second lens 112 and the third lens 113 are made of glass. Of course, other materials that can be used as lenses can also be used.

The rod lens system provided in the embodiment of this disclosure employs two lens groups, which are symmetrically arranged and have three lens combinations with specific parameters, to simultaneously correct the axial chromatic aberration of visible spectrum images and the axial chromatic aberration of near-infrared spectrum images and ensure the parfocal imaging within the range of visible light and infrared light wavelength, and avoid the problem of refocusing when observing infrared light images and white light images. At the same time, there are three types of cemented lens, which reduces the processing difficulty and cost requirements without affecting the axial chromatic aberration, while still satisfies the normal clinical requirements.

In some embodiments, the rod lens system can be applied in the visible light and near-infrared light range, and the specific application spectral range is 420nm to 900nm. That is, the wavelength range covers 420nm to 900nm, and there is no need to refocus during imaging, so it is more convenient to use.

In some embodiments, in order to improve the image transmission ability of the rod lens system, the rod lens system 10 satisfies the following requirement:

$$18 \leq T/D \leq 22 \qquad (1)$$

wherein, in formula (1), T refers to a conjugate distance of an object and image conjugate plane of the rod lens system 10, and D refers to a diameter of the elongated rod lens.

Because the main function of the rod lens system is to efficiently transmit images within a limited physical aperture. If the object and image conjugate distance of the rod lens system is too long, the numerical aperture of the light beam is bound to be reduced under the rated diameter of the lens. If the object and image conjugate distance of the rod lens system is too short, the number of the rod lens groups required within a specific physical length will increase, which affects the light transmission efficiency and cost. Therefore, formula (1) is operable to define the rod lens system, so as to improve the image transmission ability of the rod lens system and reduce the cost.

In some embodiments, in order to improve the imaging quality of the rod lens system, the first lens 111 satisfies the following requirement:

$$n_1 \leq 1.63 \qquad (2)$$

Wherein, in formula (2), $n_1$ refers to a refractive index of the first lens 111.

In addition, the first lens 111 is an elongated rod lens, and its spectral transmittance will affect the color perception of the whole lens when it is used for observation. The glass with too high refractive index has a strong blue light absorption, which makes the imaging effect of the whole lens yellow. Therefore, in terms of material selection, the elongated rod lens mostly selects low refractive index materials with high blue light transmittance.

Therefore, the first lens 111 is an elongated rod lens whose material has a low refractive index but high blue light transmittance, and its refractive index satisfies formula (2). Thus, the imaging quality can be further improved.

In some embodiments, in order to reduce the chromatic aberration of the rod lens system and broaden the use range of the spectrum, the material of the second lens 112 adopts abnormal chromatic dispersion glass, and the second lens 112 satisfies the following requirement:

$$V_2 \geq 70 \qquad (3)$$

$$\Delta P_2 \geq 0.025 \qquad (4)$$

wherein, $V_2$ refers to Abbe number of the second lens 112, and $\Delta P_2$ refers to partial chromatic dispersion of the second lens 112.

Specifically, the second lens 112 is made of abnormal chromatic dispersion glass that deviates from the P-V standard line. This is very useful for correcting the secondary spectrum, and even makes the axial chromatic aberration present an S-shaped curve, thus greatly reducing the axial chromatic aberration and broadening the application range of the spectrum.

In some embodiments, in order to improve the imaging quality, the third lens 113 satisfies the following requirement:

$$n_3 \geq 1.55 \tag{5}$$

$$V_3 \geq 40 \tag{6}$$

wherein, $n_3$ refers to a refractive index of the third lens 113, and $V_3$ refers to Abbe number of the third lens 113.

The third lens 113 is a meniscus negative lens which is adjacent to the aperture diaphragm and made of glass with a high refractive index. This is because pisvar field curvature is related to $\Sigma 1/nf$, wherein, n refers to the refractive index of the lens and f refers to the focal distance of the lens. The negative lens employing the material with high refractive index, can effectively reduce the field curvature under the condition of satisfying the overall focal power. Therefore, the refractive index $n_3$ of the third lens 113 and the Abbe number $V_3$ of the third lens 113, satisfy the requirements of formula (5) and formula (6), respectively, such that the field curvature of the rod lens system can be effectively reduced and the imaging quality of the rod lens system can be effectively improved.

In some embodiments, in order to reduce the processing difficulty of the rod lens system and improve the imaging quality of the rod lens system, a curvature radius of a lens surface of the first lens 111 which faces the diaphragm 12 is arranged to be identical as a curvature radius of a lens surface of the second lens 112 which back-faces the diaphragm 12, while a curvature radius of a lens surface of the second lens 112 which faces the diaphragm 12 is arranged to be identical as a curvature radius of a lens surface of the third lens 113 which back-faces the diaphragm 12.

Alternatively, the lens surface of the first lens 111 which back-faces the diaphragm is convex, and the lens surface of the first lens 111 which faces the diaphragm 12 is concave or flat.

Alternatively, the second lens 112 is a biconvex lens or a planoconvex lens. That is, the lens surface of the second lens 112 which back-faces the diaphragm 12 is convex or flat, while the lens surface of the second lens 112 which faces the diaphragm 12 is convex.

Alternatively, the third lens 113 is a meniscus lens.

In other embodiments, at least one piece of flat glass may be cemented between the first lens 111 and the second lens 112, or at least one piece of flat glass may be cemented between the second lens 112 and the third lens 113. Since the flat glass does not change the path of light, the rod lens structure under these embodiments can still achieve the same technical effect as the above embodiments, and the processing difficulty can be further reduced.

It can be seen that the rod lens system of the above embodiment is easy to process and low cost. At the same time, the axial chromatic aberration of visible spectrum image and the axial chromatic aberration of near-infrared spectrum image can be highly corrected at the same time. In the application of endoscope, the parfocal imaging can be guaranteed within the range of visible light and infrared light wavelength, and the problem of refocusing when observing infrared light images and white light images can be avoided, thus facilitating the clinical use.

The specific numerical configurations of several different rod lens systems and the corresponding embodiments of axial chromatic aberration effect are given in combination with the table and the attached drawings.

In each table, code n corresponds to the surface label in the rod lens system, and surface code S represents the aperture diaphragm; R (unit: mm) represents the curvature radius of each surface of the optical component; L (unit: mm) represents the thickness of the optical component or the spacing between the optical components; N (d) represents the refractive index at line d (the wavelength of line d is 588nm); V (d) represents the Abbe number on the line d, and $\triangle$P represents the partial chromatic dispersion of g light and f light. Wherein, the optical aperture D is the diameter of the elongated rod lens, the object and image conjugate distance T is the conjugate distance of the object and image conjugate surface of the rod lens system, which are both in millimeters (mm).

Wherein the surface label in the rod lens system is shown in FIG. 2. O represents the object surface, S represents the diaphragm, and I represents the image surface.

In embodiment 1, table 1 and table 2 show the specific numerical configuration of the rod lens system. The rod lens system with the numerical configuration in table 1 and table 2 is shown in FIG. 1. FIG. 3 shows the axial chromatic aberration of the rod lens system for ICG hard endoscope of embodiment 1.

Table 1

| Code n | R | L | N(d) | V(d) | ⊿P |
|---|---|---|---|---|---|
| Object | Flat surface | 6.0 | | | |
| 1 | 19.08 | 45.8 | 1.62 | 60.4 | -0.0013 |
| 2 | 29.23 | 1.7 | 1.49 | 81.6 | 0.0287 |
| 3 | -8.64 | 1.5 | 1.72 | 54.7 | -0.0081 |
| 4 | -13.21 | 0.5 | | | |
| S | Flat surface | 0.5 | | | |
| 5 | 13.21 | 1.5 | 1.72 | 54.7 | -0.0081 |
| 6 | 8.64 | 1.7 | 1.49 | 81.6 | 0.0287 |
| 7 | -29.23 | 45.8 | 1.62 | 60.4 | -0.0013 |
| 8 | -19.08 | 6 | | | |
| Image | Flat surface | | | | |

Table 2

| Conjugate distance T | 111 |
|---|---|
| Optical aperture D | 6 |

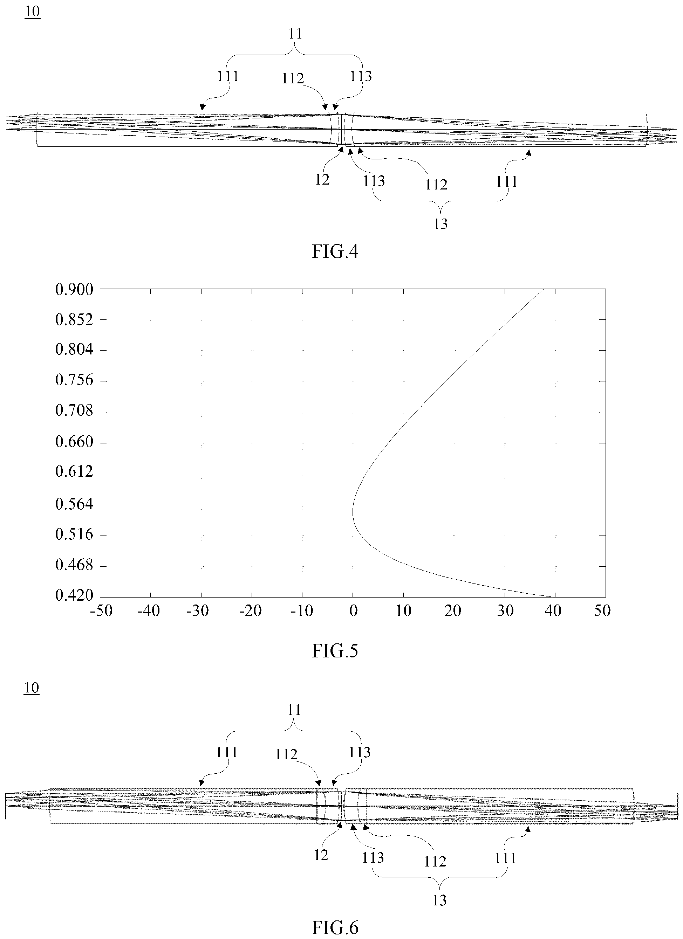

In embodiment 2, table 3 and table 4 show the specific numerical configuration of the rod lens system. The rod lens system with the numerical configuration in table 3 and table 4 is shown in FIG. 4. FIG. 5 shows the axial chromatic aberration of the rod lens system for ICG hard endoscope of embodiment 2.

Table 3

| Code n | R | L | N(d) | V(d) | ⊿P |
|---|---|---|---|---|---|
| Object | Flat surface | 5.1 | | | |
| 1 | 20.32 | 49.5 | 1.62 | 60.4 | -0.0013 |
| 2 | Infinity | 1.6 | 1.50 | 81.6 | 0.0287 |
| 3 | -9.66 | 1.4 | 1.80 | 46.6 | -0.0085 |
| 4 | -14.38 | 0.4 | | | |
| S | Flat surface | 0.4 | | | |
| 5 | 14.38 | 1.4 | 1.80 | 46.6 | -0.0085 |
| 6 | 9.66 | 1.6 | 1.50 | 81.6 | 0.0287 |
| 7 | Infinity | 49.5 | 1.62 | 60.4 | -0.0013 |
| 8 | -20.32 | 5.1 | | | |
| Image | Flat surface | | | | |

Table 4

| Conjugate distance T | 116 |
|---|---|
| Optical aperture D | 6 |

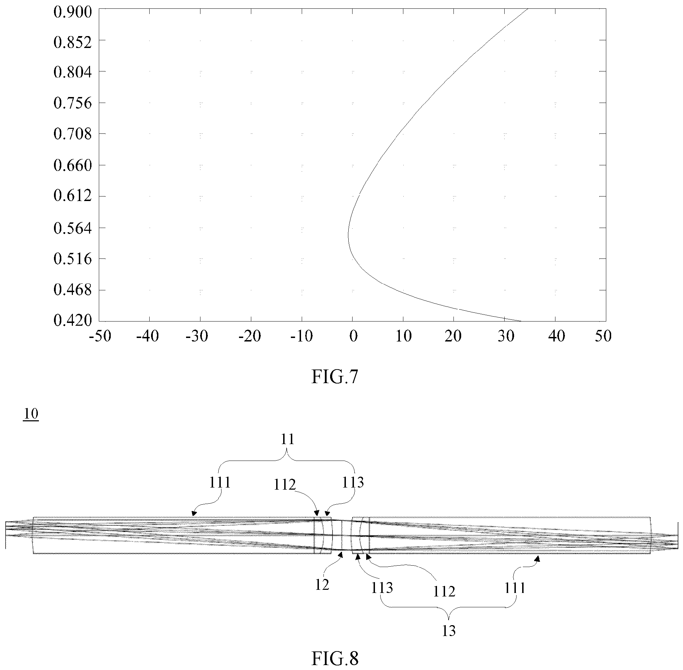

In embodiment 3, table 5 and table 6 show the specific numerical configuration of the rod lens system. The rod lens system with the numerical configuration in table 5 and table 6 is shown in FIG. 6. FIG. 7 shows the axial chromatic aberration of the rod lens system for ICG hard endoscope of embodiment 3.

Table 5

| Code n | R | L | N(d) | V(d) | $\triangle$ P |
|--------|---|---|------|------|----|
| Object | Flat surface | 7.5 | | | |
| 1 | 19.29 | 46.2 | 1.620 | 60.4 | -0.0013 |
| 2 | Infinity | 1.5 | 1.46 | 90.3 | 0.0398 |
| 3 | -9.07 | 2.3 | 1.80 | 46.6 | -0.0085 |
| 4 | -13.67 | 0.4 | | | |
| S | Flat surface | 0.4 | | | |
| 5 | 13.67 | 2.3 | 1.80 | 46.6 | -0.0085 |
| 6 | 9.07 | 1.5 | 1.46 | 90.3 | 0.0398 |
| 7 | Infinity | 46.2 | 1.62 | 60.4 | -0.0013 |
| 8 | -19.29 | 7.5 | | | |
| Image | Flat surface | | | | |

Table 6

| Conjugate distance T | 116 |
|----------------------|-----|
| Optical aperture D | 6 |

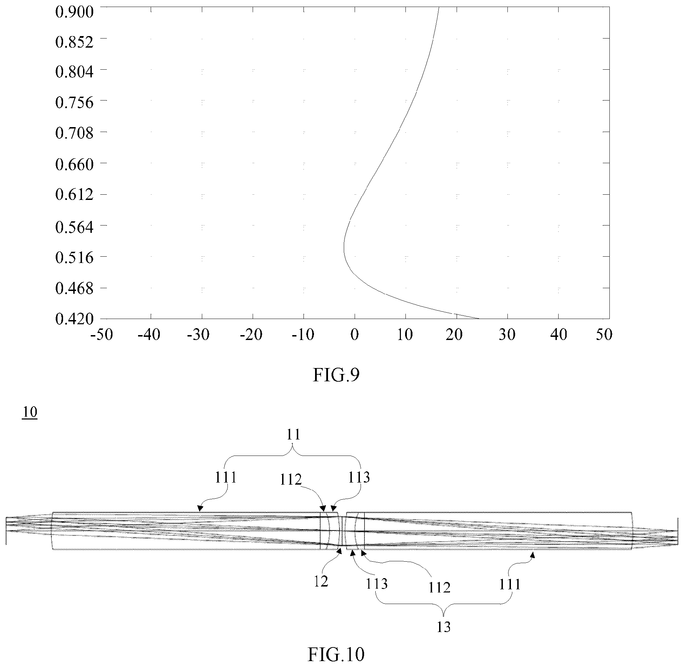

In embodiment 4, table 7 and table 8 show the specific numerical configuration of the rod lens system. The rod lens system with the numerical configuration in table 7 and table 8 is shown in FIG. 8. FIG. 9 shows the axial chromatic aberration of the rod lens system for ICG hard endoscope of embodiment 4.

Table 7

| Code n | R | L | N(d) | V(d) | $\triangle$ P |
|--------|---|---|------|------|----|
| Object | Flat surface | 4.4 | | | |
| 1 | 19.97 | 46.6 | 1.620 | 36.4 | 0.0002 |
| 2 | Infinity | 1.6 | 1.53 | 77.0 | 0.0258 |
| 3 | -7.79 | 1.5 | 1.58 | 59.6 | -0.0024 |
| 4 | -16.745 | 0.4 | | | |
| S | Flat surface | 0.4 | | | |
| 5 | 16.745 | 1.5 | 1.58 | 59.6 | -0.0024 |
| 6 | 7.79 | 1.6 | 1.53 | 77.0 | 0.0258 |
| 7 | Infinity | 46.6 | 1.62 | 60.4 | 0.0002 |
| 8 | -19.97 | 4.4 | | | |
| Image | Flat surface | | | | |

Table 8

| Conjugate distance T | 116 |
|----------------------|-----|

(continued)

| Optical aperture D | 6 |
|---|---|

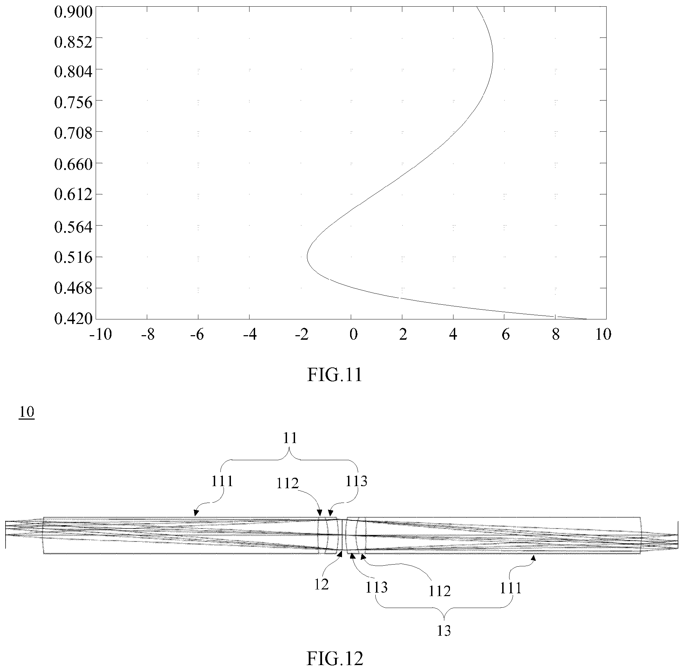

In embodiment 5, table 9 and table 10 show the specific numerical configuration of the rod lens system. The rod lens system with the numerical configuration in table 9 and table 10 is shown in FIG. 10. FIG. 11 shows the axial chromatic aberration of the rod lens system for ICG hard endoscope of embodiment 5.

Table 9

| Code n | R | L | N(d) | V(d) | △P |
|---|---|---|---|---|---|
| Object | Flat surface | 7.5 | | | |
| 1 | 18.51 | 44.4 | 1.62 | 36.4 | 0.0002 |
| 2 | Infinity | 1.5 | 1.43 | 95.2 | 0.0552 |
| 3 | -8.25 | 1.7 | 1.60 | 60.6 | -0.0003 |
| 4 | -13.58 | 0.4 | | | |
| S | Flat surface | 0.4 | | | |
| 5 | 13.58 | 1.7 | 1.60 | 60.6 | -0.0003 |
| 6 | 8.25 | 1.5 | 1.43 | 95.2 | 0.0552 |
| 7 | Infinity | 44.4 | 1.62 | 60.4 | 0.0002 |
| 8 | -18.51 | 7.5 | | | |
| Image | Flat surface | | | | |

Table 10

| Conjugate distance T | 116 |
|---|---|
| Optical aperture D | 6 |

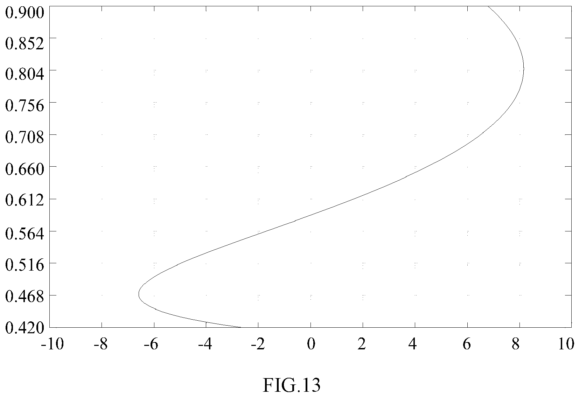

In embodiment 6, table 11 and table 12 show the specific numerical configuration of the rod lens system. The rod lens system with the numerical configuration in table 11 and table 12 is shown in FIG. 12. FIG. 13 shows the axial chromatic aberration of the rod lens system for ICG hard endoscope of embodiment 6.

Table 11

| Code n | R | L | N(d) | V(d) | △P |
|---|---|---|---|---|---|
| Object | Flat surface | 6.0 | | | |
| 1 | 17.62 | 45.5 | 1.55 | 63.5 | -0.0005 |
| 2 | 31.24 | 1.7 | 1.49 | 84.4 | 0.034 |
| 3 | -8.95 | 1.7 | 1.71 | 53.8 | -0.0083 |
| 4 | -14.00 | 0.6 | | | |
| S | Flat surface | 0.6 | | | |
| 5 | 14.00 | 1.7 | 1.71 | 53.8 | -0.0083 |
| 6 | 8.95 | 1.7 | 1.49 | 84.4 | 0.034 |
| 7 | -31.24 | 45.5 | 1.55 | 63.5 | -0.0005 |
| 8 | -17.62 | 6.0 | | | |
| Image | Flat surface | | | | |

Table 12

| Conjugate distance T | 116 |
| --- | --- |
| Optical aperture D | 6 |

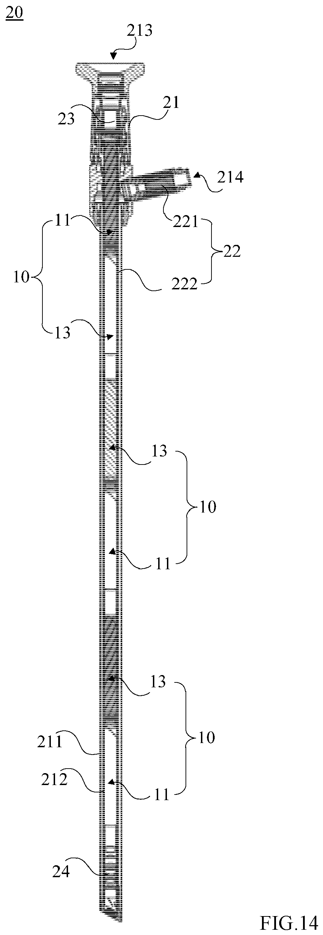

FIG. 14 is a structural diagram of an endoscope provided in another embodiment of this disclosure. Referring Fig.14, the endoscope can clearly image in the range of visible light and near-infrared light. When switching from the visible light to the near-infrared light or switching from the near-infrared light to the visible light, the endoscope can clearly image without any adjustment, which is convenient for users and satisfies the requirements of clinical medicine.

As shown in FIG.14, the endoscope 20 includes an endoscope body 21, a light guide assembly 22, an ocular lens assembly 23, an objective lens assembly 24 and a rod lens system 10.

The endoscope body 21 includes an outer sleeve 211, an inner sleeve 212, a camera interface 213 and a light source interface 214. The inner sleeve 212 is sheathed inside the outer sleeve 211. The camera interface 213 is connected with a camera device, which is operable to acquire images of an observed body. The light source interface 214 is connected with a light source device, which is operable to receive the light generated by the light source device.

The light guide assembly 22 is arranged between the outer sleeve 211 and the inner sleeve 212 and operable to guide a light, which is generated by the light source device and received through the light source interface 214, to the observed body, so as to irradiate the light on the observed body for imaging.

The ocular lens assembly 23 is arranged inside the inner sleeve 212 and adjacent to the camera interface 213. The objective lens assembly 24 is arranged inside the inner sleeve 212 and adjacent to an insertion end of the endoscope body 21. The insertion end of the endoscope body 21 refers to the head of the insertion part which is inserted into the detected target (such as a human body). The rod lens system 10 is also arranged inside the inner sleeve and is located between the ocular lens assembly 23 and objective lens assembly 24. The number of rod lens systems 10 is at least one.

It should be noted that the rod lens system 10 can be any rod lens system in the above embodiment. When there are a plurality of rod lens systems 10, of course, the plurality of rod lens systems 10 can also be a combination of the rod lens systems provided by the above different embodiments, or the plurality of rod lens systems 10 are the same rod lens system.

For example, as shown in FIG. 14, the endoscope 20 includes three rod lens systems 10. The plurality of rod lens systems 10 can effectively reduce the axial chromatic aberration, and of course, other numbers of rod lens systems can be used.

In some embodiments, the light guide assembly 22 includes a light cone 221 and an optical fiber 222. The light cone 221 is fixedly connected with the optical fiber 222, and one end of the light cone 221 corresponds to the light source interface 214 for acquiring the light generated by the light source device and coupling the light into the optical fiber 222. The optical fiber 222 is operable to guide the light to the observed body.

In some embodiments, in order to improve the imaging quality of the endoscope 20 for clearly observing the target, the ocular lens assembly 23 includes a plurality of lens combinations, and the objective lens assembly 24 may also include a plurality of lens combinations.

It can be seen that the endoscope 20 employing the rod lens system in the embodiment of this disclosure can clearly image in the range of visible light and near-infrared light. When switching from the visible light to the near-infrared light or switching from the near-infrared light to the visible light, the endoscope can clearly image without any adjustment, which is convenient for users and satisfies the requirements of clinical medicine.

Both of the outer sleeve 211 and the inner sleeve 212 are circular sleeves. When the inner sleeve 212 is sheathed inside the outer sleeve 211, the outer wall of the inner sleeve 212 can abut against the outer sleeve 211. It should be noted that the outer sleeve 211 and the inner sleeve 212 need to have a fiber channel therebetween.

It should be noted that in the endoscope 20, the diaphragm in the rod lens system 10 is a diaphragmatic diaphragm.

In other embodiments, in the rod lens system 10, at least one piece of flat glass may be cemented between the first lens and the second lens, or at least one piece of flat glass may be cemented between the second lens and the third lens. Since the flat glass does not change the path of light, the rod lens structure under these embodiments can still achieve the same technical effect as the above embodiments, and the processing difficulty can be further reduced.

The above description is only the specific embodiments of the present disclosure, and the present disclosure is not limited to this. Those skilled in the art can make various changes and modifications to the present disclosure without departing from the spirit and scope of the present disclosure. Obviously, these changes and variants should fall within the protection scope required by this present disclosure. In addition, although some specific terms are used in this disclosure, however these terms are only for convenience of explanation and do not constitute any special restrictions on this disclosure.

**Claims**

1. An endoscope, **characterized in that**, comprising:

   an endoscope body, which comprises a camera interface, a light source interface, an outer sleeve and an inner sleeve, wherein the camera interface is connected with a camera device, the light source interface is connected with a light source device, and the inner sleeve is sheathed inside the outer sleeve;
   a light guide assembly, which is arranged between the outer sleeve and the inner sleeve and operable to guide a light, which is generated by the light source device and received through the light source interface, to an observed body;
   an ocular lens assembly, which is arranged inside the inner sleeve and adjacent to the camera interface;
   an objective lens assembly, which is arranged inside the inner sleeve and adjacent to an insertion end of the endoscope body; and
   at least one rod lens system, which comprises a first lens group, a diaphragm and a second lens group which are successively arranged from an object side to an image side, wherein the first lens group and the second lens group are identical and arranged symmetrically with respect to the diaphragm; the first lens group and the second lens group each comprises a first lens, a second lens and a third lens, wherein the first lens is an elongated rod lens which has positive focal power, the second lens has positive focal power, and the third lens has negative focal power; the first lens, the second lens and the third lens are fixedly connected, the first lens is farther away from the diaphragm than the second lens and the third lens, and the third lens is closer to the diaphragm than the first lens and the second lens.

2. The endoscope according to claim 1, **characterized in that**, the light guide assembly comprises an optical fiber and a light cone which is connected with the optical fiber;
   wherein the light cone is operable to acquire the light which is generated by the light source device and couple the light into the optical fiber, and the optical fiber is operable to guide the light to the observed body.

3. The endoscope according to claim 1 or claim 2, **characterized in that**, the rod lens system is applied in a spectral range from 420nm to 900nm.

4. The endoscope according to any one of claims 1-3, **characterized in that**, the rod lens system satisfies following requirement:

$$18 \leq T/D \leq 22;$$

   wherein, T refers to a conjugate distance of an object and image conjugate plane of the rod lens system, and D refers to a diameter of the elongated rod lens.

5. The endoscope according to any one of claims 1-4, **characterized in that**, the first lens satisfies following requirement:

$$n_1 \leq 1.63 \quad ;$$

   wherein, $n_1$ refers to a refractive index of the first lens.

6. The endoscope according to any one of claims 1-5, **characterized in that**, the second lens is made of abnormal chromatic dispersion glass and the second lens satisfies following requirement:

$$V_2 \geq 70 \;, \quad \Delta P_2 \geq 0.025 \;;$$

   wherein, $V_2$ refers to Abbe number of the second lens, and $\Delta P_2$ refers to partial chromatic dispersion of the second lens.

7. The endoscope according to any one of claims 1-6, **characterized in that**, the third lens satisfies following requirement:

$$n_3 \geq 1.55 \, , \ V_3 \geq 40 \, ;$$

wherein, $n_3$ refers to a refractive index of the third lens, and $V_3$ refers to Abbe number of the third lens.

8. The endoscope according to any one of claims 1-7, **characterized in that**, a lens surface of the first lens which back-faces the diaphragm is convex, and a lens surface of the first lens which faces the diaphragm is concave or flat.

9. The endoscope according to any one of claims 1-8, **characterized in that**, the second lens is a biconvex lens or a planoconvex lens.

10. The endoscope according to any one of claims 1-9, **characterized in that**, the third lens is a meniscus lens.

11. The endoscope according to any one of claims 1-10, **characterized in that**, the endoscope further comprises at least one piece of flat glass which is arranged between the first lens and the second lens; and/or at least one piece of flat glass which is arranged between the second lens and the third lens.

12. A rod lens system, **characterized in that**, comprising a first lens group, a diaphragm and a second lens group which are successively arranged from an object side to an image side, wherein the first lens group and the second lens group are identical and arranged symmetrically with respect to the diaphragm;

the first lens group and the second lens group each comprises a first lens, a second lens and a third lens, wherein the first lens is an elongated rod lens which has positive focal power, the second lens has positive focal power, and the third lens has negative focal power;
the first lens, the second lens and the third lens are fixedly connected, the first lens is farther away from the diaphragm than the second lens and the third lens, and the third lens is closer to the diaphragm than the first lens and the second lens.

13. The rod lens system according to claim 12, **characterized in that**, the rod lens system is applied in a spectral range from 420nm to 900nm.

14. The rod lens system according to claim 12 or claim 13, **characterized in that**, the rod lens system satisfies following requirement:

$$18 \leq T/D \leq 22 \, ;$$

wherein, $T$ refers to a conjugate distance of an object and image conjugate plane of the rod lens system, and $D$ refers to a diameter of the elongated rod lens.

15. The rod lens system according to any one of claims 12-14, **characterized in that**, the first lens satisfies following requirement:

$$n_1 \leq 1.63 \qquad ;$$

wherein, $n_1$ refers to a refractive index of the first lens.

16. The rod lens system according to any one of claims 12-15, **characterized in that**, the second lens is made of abnormal chromatic dispersion glass and the second lens satisfies following requirement:

$$V_2 \geq 70 \, , \ \Delta P_2 \geq 0.025 \, ;$$

wherein, $V_2$ refers to Abbe number of the second lens, and $\Delta P_2$ refers to partial chromatic dispersion of the second lens.

17. The rod lens system according to any one of claims 12-16, **characterized in that**, the third lens satisfies following requirement:

$$n_3 \geq 1.55 \;,\;\; V_3 \geq 40 \;;$$

wherein, $n_3$ refers to a refractive index of the third lens, and $V_3$ refers to Abbe number of the third lens.

18. The rod lens system according to any one of claims 12-17, **characterized in that**, a lens surface of the first lens which back-faces the diaphragm is convex, and a lens surface of the first lens which faces the diaphragm is concave or flat.

19. The rod lens system according to any one of claims 12-18, **characterized in that**, the second lens is a biconvex lens or a planoconvex lens.

20. The rod lens system according to any one of claims 12-19, **characterized in that**, the third lens is a meniscus lens.

21. The rod lens system according to any one of claims 12-20, **characterized in that**, the endoscope further comprises at least one piece of flat glass which is arranged between the first lens and the second lens; and/or at least one piece of flat glass which is arranged between the second lens and the third lens.

10

11

111          112          113

12   113   112   111

13

FIG.1

10

1          2   S   5   6                    I

O          3   4   7                    8

FIG.2

FIG.3

FIG.4

FIG.5

FIG.6

FIG.7

FIG.8

FIG.9

FIG.10

FIG.11

FIG.12

FIG.13

FIG.14

## INTERNATIONAL SEARCH REPORT

International application No.

**PCT/CN2019/111324**

**A. CLASSIFICATION OF SUBJECT MATTER**

A61B 1/00(2006.01)i;  G02B 13/00(2006.01)i

According to International Patent Classification (IPC) or to both national classification and IPC

**B. FIELDS SEARCHED**

Minimum documentation searched (classification system followed by classification symbols)

A61B:; G02B

Documentation searched other than minimum documentation to the extent that such documents are included in the fields searched

Electronic data base consulted during the international search (name of data base and, where practicable, search terms used)

CNPAT, CNKI, WPI, EPODOC: 棒, 杆, 凸, 正, 凹, 负, 透镜, 对称, 相同, 镜像, 光闸, 光澜, 光栏, 光瞳, 内窥镜, 内视镜, 内诊镜, 内镜, 中继, 转像, rod, lens??, stop, aperture, iris, diaphragm?, endoscop+

**C. DOCUMENTS CONSIDERED TO BE RELEVANT**

| Category* | Citation of document, with indication, where appropriate, of the relevant passages | Relevant to claim No. |
|---|---|---|
| Y | CN 102004309 A (ZHANG, Yangde) 06 April 2011 (2011-04-06)<br>     description, paragraphs [0023]-[0038], and figures 1-8 | 1-11 |
| Y | CN 109124544 A (AVATERAMEDICAL GMBH) 04 January 2019 (2019-01-04)<br>     description, paragraphs [0031]-[0042], and figures 1-3 | 1-11 |
| X | CN 109124544 A (AVATERAMEDICAL GMBH) 04 January 2019 (2019-01-04)<br>     description, paragraphs [0031]-[0042], and figures 1-3 | 12-21 |
| A | CN 105445926 A (TIANJIN RONGHE ELECTRICAL TECHNOLOGY CO., LTD.) 30<br>March 2016 (2016-03-30)<br>     entire document | 1-21 |
| A | CN 105377113 A (NOVADAQ TECHNOLOGIES INC.) 02 March 2016 (2016-03-02)<br>     entire document | 1-21 |
| A | CN 103969789 A (BEIJING WESTON ASIA PACIFIC OPTO OELECTRIC<br>INSTRUMENTS CO., LTD.) 06 August 2014 (2014-08-06)<br>     entire document | 1-21 |
| A | CN 107085295 A (JIANGSU (CHINA) EAGLESCOPE MEDICAL EQUIPMENT LIMITED<br>COMPANY) 22 August 2017 (2017-08-22)<br>     entire document | 1-21 |

☑ Further documents are listed in the continuation of Box C.       ☑ See patent family annex.

| | |
|---|---|
| *      Special categories of cited documents:<br>"A"   document defining the general state of the art which is not considered<br>       to be of particular relevance<br>"E"    earlier application or patent but published on or after the international<br>       filing date<br>"L"    document which may throw doubts on priority claim(s) or which is<br>       cited to establish the publication date of another citation or other<br>       special reason (as specified)<br>"O"   document referring to an oral disclosure, use, exhibition or other<br>       means<br>"P"    document published prior to the international filing date but later than<br>       the priority date claimed | "T"    later document published after the international filing date or priority<br>       date and not in conflict with the application but cited to understand the<br>       principle or theory underlying the invention<br>"X"    document of particular relevance; the claimed invention cannot be<br>       considered novel or cannot be considered to involve an inventive step<br>       when the document is taken alone<br>"Y"    document of particular relevance; the claimed invention cannot be<br>       considered to involve an inventive step when the document is<br>       combined with one or more other such documents, such combination<br>       being obvious to a person skilled in the art<br>"&"    document member of the same patent family |

| Date of the actual completion of the international search | Date of mailing of the international search report |
|---|---|
| **23 June 2020** | **15 July 2020** |

| Name and mailing address of the ISA/CN | Authorized officer |
|---|---|
| **China National Intellectual Property Administration (ISA/<br>CN)**<br>**No. 6, Xitucheng Road, Jimenqiao Haidian District, Beijing<br>100088**<br>**China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2019/111324**

| C. | DOCUMENTS CONSIDERED TO BE RELEVANT | |
|---|---|---|
| Category* | Citation of document, with indication, where appropriate, of the relevant passages | Relevant to claim No. |
| A | CN 104040408 A (OLYMPUS WINTER & IBE GMBH) 10 September 2014 (2014-09-10) entire document | 1-21 |
| A | US 6490085 B1 (RICHARD WOLF G.M.B.H.) 03 December 2002 (2002-12-03) entire document | 1-21 |

## INTERNATIONAL SEARCH REPORT
### Information on patent family members

| International application No. |
| --- |
| **PCT/CN2019/111324** |

| Patent document cited in search report | | | Publication date (day/month/year) | Patent family member(s) | | | Publication date (day/month/year) |
| --- | --- | --- | --- | --- | --- | --- | --- |
| CN | 102004309 | A | 06 April 2011 | CN | 102004309 | B | 23 May 2012 |
| CN | 109124544 | A | 04 January 2019 | RU | 2018121614 | A | 16 December 2019 |
| | | | | CA | 3007253 | A1 | 16 December 2018 |
| | | | | EP | 3417761 | A1 | 26 December 2018 |
| | | | | JP | 2019032509 | A | 28 February 2019 |
| | | | | EP | 3417761 | B1 | 08 April 2020 |
| | | | | DE | 102017113271 | A1 | 20 December 2018 |
| | | | | US | 2018364473 | A1 | 20 December 2018 |
| CN | 105445926 | A | 30 March 2016 | | None | | |
| CN | 105377113 | A | 02 March 2016 | KR | 20170129282 | A | 24 November 2017 |
| | | | | US | 2014343362 | A1 | 20 November 2014 |
| | | | | JP | 6224228 | B2 | 01 November 2017 |
| | | | | KR | 20160007611 | A | 20 January 2016 |
| | | | | US | 9918619 | B2 | 20 March 2018 |
| | | | | HK | 1222525 | A1 | 07 July 2017 |
| | | | | JP | 2018049275 | A | 29 March 2018 |
| | | | | CA | 2911861 | A1 | 18 December 2014 |
| | | | | WO | 2014199236 | A2 | 18 December 2014 |
| | | | | EP | 2996543 | A2 | 23 March 2016 |
| | | | | CN | 105377113 | B | 16 November 2018 |
| | | | | KR | 101799545 | B1 | 20 November 2017 |
| | | | | CA | 2911861 | C | 03 September 2019 |
| | | | | JP | 2016519341 | A | 30 June 2016 |
| CN | 103969789 | A | 06 August 2014 | | None | | |
| CN | 107085295 | A | 22 August 2017 | WO | 2019001275 | A1 | 03 January 2019 |
| CN | 104040408 | A | 10 September 2014 | WO | 2013102476 | A1 | 11 July 2013 |
| | | | | US | 9817227 | B2 | 14 November 2017 |
| | | | | US | 2014313578 | A1 | 23 October 2014 |
| | | | | JP | 2015508511 | A | 19 March 2015 |
| | | | | CN | 104040408 | B | 26 April 2017 |
| | | | | JP | 5934383 | B2 | 15 June 2016 |
| | | | | DE | 102012200146 | A1 | 11 July 2013 |
| US | 6490085 | B1 | 03 December 2002 | | None | | |

Form PCT/ISA/210 (patent family annex) (January 2015)